# EUROPEAN PATENT SPECIFICATION

(11) **EP 1 878 453 B1**
(45) Date of publication and mention of the grant of the patent: **24.12.2014**
(21) Application number: 06745656.6
(22) Date of filing: 25.04.2006
(51) Int. Cl.: A61M 1/12, A61M 25/00, A61M 1/10

(54) **INTRA-AORTIC BALLOON PUMPING SET**
INTRAAORTALER BALLONPUMPSATZ
ENSEMBLE DE POMPAGE A BALLONNET INTRA-AORTIQUE

(30) Priority: 26.04.2005 JP 2005128639
(43) Date of publication of application: 16.01.2008
(73) Proprietor: Zeon Corporation, Tokyo 100-8246 (JP)
(72) Inventor: MORI, Kenji, ZEON CORPORATION, Chiyoda-ku, Tokyo 100-8246 (JP); OOKAWA, Yasuhiro, ZEON CORPORATION, Tokyo 1008246 (JP); IIDA, Takahiro, ZEON CORPORATION, Tokyo 1008246 (JP)
(74) Representative: Bertsch, Florian Oliver
(86) International application number: PCT/JP2006/308628
(87) International publication number: WO 2006/118103

(56) References cited:
- EP-A2- 0 992 260
- JP-A- 06 165 821
- JP-A- 2000 107 293
- JP-A- 2001 231 865
- US-A- 4 697 573
- US-A- 5 460 607
- US-A- 5 480 383
- US-A- 5 711 754
- US-A1- 2003 032 974

## Description

### TECHNICAL FIELD

The present invention relates to an intra-aortic balloon pumping set used for treatment by intra-aortic balloon pumping method to increase bloodstream in coronary artery, etc. of a patient by inserting an intra-aortic balloon catheter in the aorta and inflating and deflating the balloon. More particularly, the present invention relates to an intra-aortic balloon pumping set, capable to reduce a burden on a patient and to inflate and deflate the balloon with a high response.

### BACKGROUND ART

As a method for treatment at the time of deterioration of heart functions, the intra-aortic balloon pumping method (hereinafter may be referred to as "IABP" method), to support heart functions by inserting a balloon catheter in the aorta and inflating and deflating the balloon in accordance with the heartbeat to improve blood pressure in the aorta, is known.

In the IABP method, it is necessary to figure out a state of a patient' s heartbeat by electrical signal generated at sinus node, etc. and to inflate and deflate the balloon by introducing pressurized fluid into the balloon and guiding it out in accordance with the heartbeat. Thus, in a balloon catheter used for IABP method, a balloon has to be inflated and deflated in accordance with the heartbeat even having very short cycle (namely, high response) . It is known that the response of the balloon catheter depends on a flow channel of pressurized fluid used for inflating and deflating the balloon, and cross-sectional area of the pressurized fluid may be enlarged in order to obtain good response.

However, since most of the flow channel of the pressurized fluid is positioned in a catheter tube of the balloon catheter, simple increase in the cross-sectional area of the pressurized fluid makes the catheter tube thicker, so that burden on a patient is increased when inserting the catheter tube into a body. Therefore, as a method for solving this problem, there has been developed an art that a portion of a distal end of the catheter tube inserted into a blood vessel is made narrower to decrease a burden on a patient and a portion of a proximal end not inserted into the blood vessel is made thick to maintain cross-sectional area of the pressurized fluid (e.g., refer to US Patent No. 5460607 corresponding to JP Patent Publication No. 6-165821 and JP Patent Publication No. 2001-231865).

In recent years, however, it is more required to reduce a burden on a patient in a variety of treatments. A balloon catheter used in IABP method is also required to further reduce a burden on a patient by reducing the diameter thereof, so that the method in the above-mentioned patent literature becomes insufficient in improved effects for response. Accordingly, an art is strongly desired to maintain the response sufficiently even if reducing the diameter of a balloon catheter for reducing a burden of patient.

### DISCLOSURE OF INVENTION

### PROBLEM TO BE SOLVED BY THE INVENTION

The present invention was made in consideration of this situation and an object is to provide an intra-aortic balloon pumping set capable to reduce the patient burden and to inflate and deflate the balloon for treatment by IABP method with a good response.

### MEANS FOR SOLVING THE PROBLEM

The present inventors, as a result of keen examinations, have completed the present invention based on the findings that it attains the above object to provide an introducer used for inserting a catheter to blood vessel and an intra-aortic balloon catheter, comprising an outer tube having a specific shape corresponding to a dimension of a determined portion of the introducer, as a set.

Namely, according to the present invention, there is provided an intra-aortic balloon pumping set comprising an intra-aortic balloon catheter having a balloon portion insertable inside the aorta and inflating and deflating to assist the heart function, an outer tube with a distal end connected to a proximal end of the balloon portion to introduce pressurized fluid in said balloon portion and guide it out, and an inner tube, to which a distal end of said balloon portion is connected, extending through the insides of said balloon portion, and said outer tube in the axial direction, and
an introducer used for inserting said balloon portion into a blood vessel, wherein said outer tube comprises a first portion, a second portion continuously provided at the proximal end of the first portion and a third portion continuously provided at the proximal end of the second portion;
said introducer has a configuration of cylindrical shape into which a tubular member having an outer diameter of B (mm) or smaller can be inserted;
an outer diameter of said first portion is less than or equal to A(mm) and a length in the axial direction of said first portion is 1 to 400 (mm) ;
an outer diameter of said second portion is A(mm) or larger and less than B(mm) and a length in the axial direction of said second portion is C-10 (mm) to C+30 (mm); and
an outer diameter of said third portion is larger than B(mm); while an inner diameter of an distal end of said introducer is A (mm), and a length in the axial direction of said introducer is C (mm) .

It is preferable that the inner diameter of said second portion is larger than the inner diameter of said first portion, and that the inner diameter of said third portion is larger than the inner diameter of said second portion.

Also, it is preferable that differences in diameters are provided at the boundary portion between said first portion and said second portion and the boundary portion between said second portion and said third portion, respectively, and that the outer diameter of said second portion is substantially uniform in the axial direction.

Further, the length of said third portion is 1 to 70 (mm).

Preferably, the outer diameter of said first portion is 90% or larger and less than 100% of A(mm) which is the inner diameter of the distal end of said introducer.

According to the intra-aortic balloon pumping set of the present invention, when providing the IABP treatment, a burden on a patient can be reduced and a balloon can be inflated and deflated with a high response.

### BRIEF DESCRIPTION OF DRAWINGS

[Fig. 1] Fig. 1 is a schematic view of an embodiment of an intra-aortic balloon catheter of an intra-aortic balloon pumping set of the present invention.
[Fig. 2] Fig. 2 is a schematic view of an embodiment of the introducer of the intra-aortic balloon pumping set of the present invention and a dilator used in combination therewith.

### BEST MODE FOR CARRYING OUT THE INVENTION

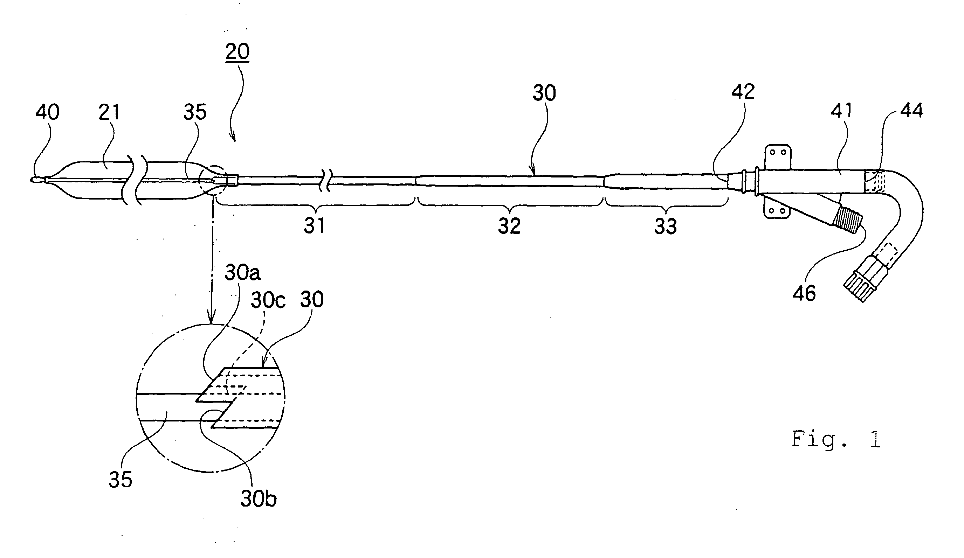

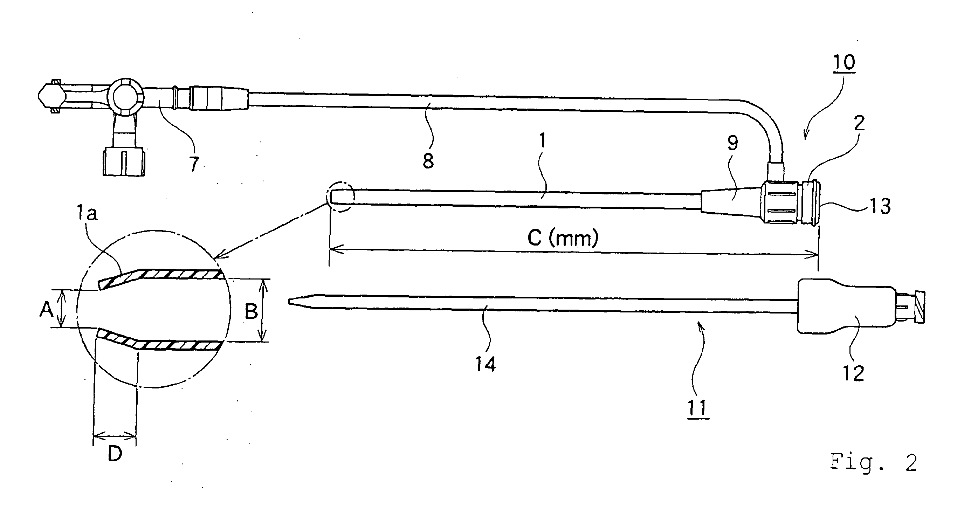

Below, the present invention will be described based on embodiment shown in the drawings.
The intra-aortic balloon pumping (IABP) set of the present invention is used for IABP method, and comprises the intra-aortic balloon catheter 20 shown in Fig. 1 and the introducer 10 shown in Fig. 2. The balloon catheter 20 shown in Fig. 1 comprises a balloon portion 21 which inflates and deflates to assist the heart function. The introducer 10 shown in Fig. 2 is used to insert the balloon catheter 20 shown in Fig. 1 into a blood vessel.

The introducer, which is a component of the intra-aortic balloon pumping set of the present invention, comprises a sheath having the distal end and the proximal end. A structure of the introducer of the present invention is not particularly limited as far as it is used to insert the intra-aortic balloon catheter into a blood vessel via a lumen of the sheath 1 with inserting a distal end of the sheath into a blood vessel from external side of a patient. Below, the introducer 10 shown in Fig. 2 will be explained as an embodiment of the introducer used in the present invention.

The introducer 10 of an embodiment shown in Fig. 2 comprises a sheath 1 as a tubular member having a distal end and a proximal end, a sheath hub 2 connected to the proximal end of the sheath 1, a strain relief 9 externally fitted to a connecting portion of the sheath 1 and the sheath hub 2 and a side infusion tube 8 connected to a side portion of the sheath hub 2.

The sheath 1 is composed of a cylindrical tube formed by polymer material. As a polymer material, although not particularly limited, for example, there may be mentioned polyolefine such as polyethylene and polypropylene; polyamide such as nylon 12; polyester such as polyamide elastomer, polyurethane, polyethylene terephthalate and polybutylene terephthalate; polyester elastomer; and fluorine-based resin.

Note that radiopaque material such as bismuth oxide and barium sulfate may be blended to materials of the sheath 1 so as to confirm a position of the sheath 1 inserted into a body by X-ray illumination. Also, at outer circumferential face of the sheath 1, hydrophilic polymer material such as polyethylene oxide may be coated to facilitate insert and removal of the sheath 1.

An inner diameter of the tube constituting the sheath 1 is 1.51 to 4.20mm, and particularly preferably 2.01 to 3.20mm. If the diameter is too small, an outer tube 30 of the intra-aortic balloon catheter 20 shown in Fig. 1 (a second portion 32 mentioned later) becomes narrower, response properties of the balloon portion 21 is liable to be insufficient. Also, when an inner diameter of tube constituting the sheath 1 is too large, an outer diameter of the sheath 1 becomes larger, so that a burden of a patient to whom the sheath 1 is inserted becomes larger.

A thickness of the tube constituting the sheath 1 is preferably 0.03 to 0.30mm. When the thickness is too small, strength of the sheath 1 is liable to become insufficient, when too large, a burden of a patient to whom the sheath 1 is inserted becomes larger since the outer diameter of the sheath 1 becomes large. Also, a length of the sheath 1 is normally 30 to 500mm and preferably 100 to 300mm. When the length of the sheath 1 is too short, it will be difficult to insert the sheath 1 into a blood vessel of the patient, and to maintain the inserted state of the introducer 10 into the patient, so that a long period treatment by IABP method is liable to be difficult. Further, when the length of the sheath 1 is too long, it is liable to be difficult to handle the introducer 10.

A distal end portion 1a of the sheath 1 is normally tapered shape, wherein the distal end becomes narrower to its end, so as to easily insert the introducer 10 when inserting with a dilator 11 into a body without difficulty as described below. Preferably, in this tapered shape, an inner diameter A of the distal end of the sheath 1 is smaller by 0.01 to 0.20mm than an inner diameter B of the tube constituting the sheath 1. It is also preferable that the inner diameter A of the distal end of the sheath 1 is 1.50 to 4.00mm, and particularly preferably 2.00 to 3.00mm is. When the inner diameter is too small, an outer tube 30 (a first portion 31 as described below) of the intra-aortic balloon catheter 20 becomes narrower, so that a response of the balloon portion 21 is liable to be insufficient. When too large, a burden given to a patient is liable to become larger when inserting the sheath 1 into a body.

Note that an axial length D of the tapered shape portion of the distal end portion 1a of the sheath 1 is, although not particularly limited, preferably 0.5 to 5.0mm.

In the introducer 10 of an embodiment shown in Fig. 2, a sheath hub 2 is connected to the proximal end of the sheath 1. The sheath hub 2 is an about cylindrical member internally having a cavity connecting through a lumen of the sheath 1 . As a material forming the sheath hub 2, although not particularly limited, there may be mentioned, for example, polyolefine such as polyethylene and polypropylene, high-polymer materials such as polyamide, polycarbonate and polystyrene.

In the cavity of the sheath hub 2, normally, the intra-aortic balloon catheter 20 and the dilator 11 can be inserted, a haemostatic valve (not shown) is provided to prevent blood passage regardless of insertion of the intra-aortic balloon catheter 20 etc. Thereby, blood leakage out of the body though the introducer 10 can be prevented. As the haemostatic valve, normally, a valve body composed by providing a slit penetrating both faces of a plate like body formed by silicone rubber, butyl rubber, isoprene rubber etc. is used.

In the introducer 10 of the present embodiment, a ring shape cap 13 is fitted at a distal end side of the sheath hub 2 wherein the haemostatic valve is placed. This cap 13 is fitted to the sheath hub 2 in order to hold the haemostatic valve in the cavity of the sheath hub 2 while making possible to insert the intra-aortic balloon catheter 20 into the slit of the haemostatic valve.

Note that it is possible to say that members capable to be inserted into the slit of the haemostatic valve can be inserted to the introducer 10 internally in the introducer 10 of the present embodiment.

At a side face of the sheath hub 2 of the introducer 10 of the present embodiment, a branch pipe communicating between the cavity in the sheath hub 2 and outside is formed. To the branch pipe, one end of the side infusion tube 8 composed of a transparent elastomer is connected. At the other end of the side infusion tube 8, a three-way valve is mounted. The side infusion tube 8 can be used for purposes of injecting medical solution such as heparin in to a patient's blood vessel, and connecting a blood pressure monitor, etc.

In the introducer 10 of the present embodiment, a strain relief 9 is fitted outwardly on an outer circumferential face of a connecting portion of the sheath 1 and the sheath hub 2. The strain relief 9 is, for example, composed of thermal plastic elastomer, such as styrene elastomer, olefinic elastomer and polyester elastomer. This strain relief 9 is a tubular body having an about conical shape and has a function to prevent kink (a phenomenon of permanent set due to bending) of the sheath 1 near the connecting portion with the sheath hub 2.

When inserting the introducer 10 into the patient body, the dilator 11 shown in Fig. 2 is normally used. The dilator 11 comprises a dilator body 14, which is a tubular body having a tapered distal end, and a dilator hub 12 attached to a proximal end of the dilator body 14.

The dilator hub 12 has a connectable structure with the sheath hub 2 . When inserting the dilator body 14 from a proximal end side of the introducer 10 and connecting the dilator hub 12 and the sheath hub 2, the tapered portion of the dilator body 14 is projected from the sheath 1, and an edge of the dismal end of the sheath 1 is closely attached to an outer circumferential face of the dilator body 14.

As stated above, when using the introducer 10 and the dilator 11 in combination to needle the tapered portion of the dilator body 14 along with a guide wire to the patient, a puncture hole is gradually stretched, so that it is possible to insert the introducer 10 into the patient body by continuing the insertion. By removing the dilator 11 after the sheath 1 of the introducer 10 reaches into the blood vessel of the patient, it becomes possible to insert the intra-aortic balloon catheter 20 into the patient' s blood vessel via the introducer 10. Note that the dilator body 14 of the dilator 11 is formed by, for example, same materials of the above stated sheath 1, and the dilator hub 12 is formed by, for example, same materials of the above stated sheath hub 2.

As shown in Fig. 1, the intra-aortic balloon catheter 20 as a component of the intra-aortic balloon pumping set of the present invention comprises the balloon portion 21, the outer tube 30 and the inner tube 35. The balloon portion 21 is inserted inside the aorta, and inflates and deflates to assist the heart function. The distal end of the outer tube 30 is connected to the proximal end of the balloon portion 21 to introduce and lead out pressurized fluid inside the balloon portion 21. The distal end of the balloon portion 21 is connected to the distal end of the inner tube 35 to extend through the insides of the balloon portion 21 and the outer tube 30 in the axial direction.

The outer tube 30 comprises a certain structure corresponding to a dimension of a specific portion of the introducer 10. Namely, the outer tube 30 comprises a certain structure corresponding to the inner diameter [A(mm)] of the distal end of the sheath 1 of the introducer 10, a possible maximum value [B(mm)] of the outer diameter of the tubular member capable to be inserted into an internal portion of the introducer 10, and an axial length [C(mm)] of the introducer 10.

Note that the inner diameter is measured by pin gauges having scales per 0.01mm in the present invention. Pin gauges are inserted into the distal end of the sheath 1 in increasing order of the diameter to determine the distal end inner diameter, defined as 0.01mm smaller numerical value than a value of the gauge not movable by pushing with one index finger. The outer diameter is also measured by ring gauges.

Note that "being able to insert into an internal portion of the introducer 10" indicates that it is possible to insert into the introducer 10 without breakage. Therefore, for example, when the haemostatic valve is broken and no more carries out its function due to the inserted tubular member, it cannot be said "being able to insert". Hereinafter, the intra-aortic balloon catheter 20 shown in Fig. 1 will be explained as an embodiment of the intra-aortic balloon catheter used in the present invention.

The intra-aortic balloon catheter 20 as an embodiment shown in of Fig. 1 comprises an end tip 40 and a connector 41. The end tip 40 is connected to the distal end portion of the balloon portion 21. The connector 41 is connected to the proximal end portion of the outer tube 30.

The balloon portion 21 of the intra-aortic balloon catheter 20 is a tubular body which is composed of an inflation deflation portion to be inflated and deflated by introducing and leading out fluid via the inner portion of the outer tube 30, a distal end portion positioned at a distal end side of the inflation and deflation portion, and a proximal end portion positioned at a proximal end side of the inflation and deflation portion.

The distal end portion and the proximal end portion of the balloon portion 21 are used as fitting overlap widths in order to fit the balloon portion 21 to the distal end portion of the outer tube 30, which are not inflating and deflating by a fluid. At the inflation and the deflation portions of the balloon portion 21, there are provided a distal end side tapered portion and a proximal end side tapered portion, extending in tapered shapes to taper toward the distal end portion and the proximal end portion.

An outer diameter and a length of the inflating and deflating portion of the balloon portion 21 is determined according to an inner volume of the inflating and deflating portion having a large impact on assisting heart functions and an inner diameter of an artery, etc. For example, when the inner volume of the inflating and deflating portion is 20 to 50cc, preferably, the outer diameter is 12 to 20mm and an axial length is 150 to 270mm. Also preferably, a membrane thickness of the inflating and deflating portion is 30 to 120µm. Preferably, inner diameters of the distal end portion and the proximal end portion of the balloon portion 21 are substantially equal to outer diameters of the inner tube 35 and the outer tube 30 to which the distal end portion and the proximal end portion are connected respectively, and are normally within a range of 0.5 to 3.4mm. Also preferably, lengths of the distal end portion and the proximal end portion are 3 to 15mm.

A material of the balloon portion 21 is, although not particularly limited, preferably material having excellent antithrombogenicity and resistance to flexibility fatigue. For example, it is composed of synthetic resin, such as urethane based elastomer and copolymer of polyurethane and silicon. As a method to form the balloon portion 21, although not particularly limited, dip-forming method and a blow molding method are preferably used.

The inner tube 35 of the intra-aortic balloon catheter 20 is a tubular body in which a lumen is internally formed along with axial direction. The inner tube 35 axially extends in the lumen of the outer tube 30, and both ends of the inner tube 35 project from the outer tube 30. The lumen of the inner tube 35 can be used as a blood flow channel for blood pressure taking and also used for putting through a guide wire used when inserting the intra-aortic balloon catheter 20 into the artery of a patient.

The inner diameter of the inner tube 35 is not particularly limited as far as it is possible to put through the guide wire, and is preferably 0.1 to 1.5mm. The thickness of the inner tube 35 is not particularly limited as far as it is possible to support the balloon portion 21, but is preferably 0.05 to 0.4mm.

Also, the axial length of the inner tube 35 is, normally, 550 to 1100mm. As a material to constitute the inner tube 35, metal, synthetic resin or composite material wherein synthetic resin is strengthen by metal, having high bending elastic modulus as well as a certain level of flexibility, are preferably used. As a metal, there may be mentioned stainless, tungsten, nickel-titanium alloy, etc. Also as a synthetic resin, polyether ether ketone etc., are exemplified. In view of having higher elasticity and resistance to fatigue according to vibration due to inflating and deflating of the balloon portion 21, using polyether ketone is preferable among them.

The outer tube 30 of the intra-aortic balloon catheter 20 is a tubular body internally having a lumen in an axial direction. The lumen of the outer tube 30 is utilized as a channel of fluid for inflating and deflating the balloon portion 21. The shorter a length of the channel is, or the larger a flow cross sectional area is, the smaller a flow channel resistance becomes, so that a response of the balloon portion 21 becomes favorable.

In the intra-aortic balloon catheter 20 of an embodiment shown in Fig. 1, the outer tube 30 comprises a first portion 31 at the most distal end side, a second portion 32 continuing into a proximal end side of the first portion 31, and a third portion 33 continuing into an proximal end side of the second portion 32. These portions 31 to 33 comprise different outer diameters respectively so that the outer tube 30 becomes larger in the proximal end side.

The first portion 31 of the outer tube 30 of the intra-aortic balloon catheter 20 is a portion positioned at the most distal end side portion of the outer tube 30 that has an outer diameter smaller than a distal end inner diameter of the sheath 1 of the introducer 10 provided as a component of the intra-aortic balloon pumping set. Namely, the first portion 31 of the outer tube 30 comprises an outer diameter less than the inner diameter A(mm) of the distal end of the sheath 1 of the introducer 10.

Therefore, the first portion 31 passes the distal end opening of the sheath 1 to be placed in a blood vessel of a patient when the outer tube 30 is inserted into the introducer 10 punctured the patient. An axial length of the first portion 31 is required to be 1 to 400mm, and is preferably 200 to 300mm. When the length of the first portion 31 is too short, it becomes difficult to keep the balloon portion 21 inside the aorta of the patient; when too longer, it is liable to deteriorate the response of the balloon portion 21 because a flow channel resistance of a pressurize fluid becomes higher.

Although an outer diameter of the first portion 31 of the outer tube 30 is not particularly limited as far as it is less than A(mm), it is preferable to maximize within a range less than A(mm) in view of improving the response of the balloon portion 21. Specifically, it is preferably 90% or larger and less than 100%, and particularly preferably, 95% or larger and 99.99% or smaller. Note that although a taper can be provided at the first portion 31 of the outer tube 30, in view of improving the response of the balloon portion 21, the first portion 31 is preferably a straight cylindrical shape.

The second portion 32 of the outer tube 30 of the intra-aortic balloon catheter 20 is a portion continuing into the proximal end of the first portion 31. The second portion 32 comprises an outer diameter equal to or larger than the inner diameter of a distal end of the sheath 1 of the introducer 10 provided as a component of the intra-aortic balloon pumping set. In addition, the second portion 32 comprises an outer diameter equal to or less than the maximum value of an outer diameter of a tubular body, which is able to be inserted into the introducer 10. Namely, the second portion is a portion having an outer diameter of A to B(mm).

Therefore, the second portion 32 can be inserted internally into the introducer 10 when inserting the outer tube 30 into the introducer 10 punctured the patient, but cannot pass a distal end opening of the sheath 1. Preferably, an axial length of the second portion 32 is not shorter exceeding 10mm than an axial length C of the introducer 10, and is not longer exceeding 30mm than an axial length of the introducer 10.

Namely, the axial length of the second portion 32 is preferably (C-10) to (C+30)(mm) and more preferably (C-5) to (C+20)(mm). When the length of the second portion 32 is too short to the axial length C of the introducer 10, the first portion 31 is consequently required to make longer, and flow channel resistance of pressurized fluid becomes higher, so that it is liable to deteriorate the response of the balloon portion 21. Further, when the length of the second portion 32 is too long to the axial length of the introducer 10, it is liable to deteriorate the response of the balloon portion 21.

A shape of the second portion 32 of the outer tube 30 is not particularly limited as far as its outer diameter is within a range of A to B(mm) . As a specific shape of the second portion 32, a straight cylindrical shape, or a tapered shape as a whole are exemplified. Also, it is possible to be a shape having the largest possible outer diameter at respective portions in response to an internal shape of the introducer 10. However considering the productivity of the outer tube 30, the shape of the second portion 32 is preferably a straight cylindrical shape, and the outer diameter of the second portion 32 in this case is preferably 102 to 110% of the dimension A(mm).

The third portion 33 of the outer tube 30 of the intra-aortic balloon catheter 20 is a portion continuing into the proximal end of the second portion 32 and is a portion having an outer diameter exceeding a maximum possible value of an outer diameter of a tubular body which is able to be inserted into the introducer 10 provided as a component of the intra-aortic balloon pumping set. Namely, the third portion 33 comprises an outer diameter exceeding a dimension B (mm). Therefore, the third portion 33 cannot be inserted into the introducer 10. An axial length of the third portion 33 is preferably 1 to 70mm and more preferably 20 to 50mm.

A shape of the third portion 33 of the outer tube 30 is not particularly limited as far as its outer diameter exceeds B (mm) . As a specific shape of the third portion 33, a straight cylindrical shape, or a tapered shape are exemplified. However, considering the productivity of the outer tube 30, the shape of the third portion 33 is preferably a straight cylindrical shape.

A thickness of the outer tube 33 is preferably 0.05 to 0.3mm and more preferably 0.05 to 0.15mm in the first to third portions 31 to 33. Also, the thickness of the outer tube 33 may be same or different in the first to third portions 31 to 33, respectively. However, in case that the thickness of the outer tube 30 is different, it is preferable to set each thickness so that the inner diameter of the second portion 32 is larger than the inner diameter of the first portion 31, and that the inner diameter of the third portion 33 is larger than the inner diameter of the second portion 32 . Note that the axial length of the whole outer tube 30 is, normally, 400 to 800mm:

Further, in respective boundary portions of the first to third portions 31 to 33 of the outer tube 30, steps may be formed and taper may be formed. However, in view of maintaining a larger flow channel cross sectional area of the pressurized fluid, it is preferable to form steps. As a specifically preferable construction of the outer tube 30, there may be a construction that steps are respectively formed at a boundary portion of the first portion 31 and the second portion 32, and a boundary portion of the second portion 32 and the third portion 33 in the outer circumference of the outer tube 30, and that the second portion 32 comprises substantially uniform outer diameter in the axial direction of the outer tube 30.

A material constituting the outer tube 30 is, although not particularly limited, for example, a synthetic resin such as polyethylene, polypropylene, polyethylene terephthalate, polyamide, polyvinyl chloride, polyurethane and fluorine contained resin is preferable. Among them, polyamide is preferable.

A forming method of the outer tube 30 is not particularly limited, too. The following method is mentioned as an instant thereof. Namely, a metallic mandrel comprising three types of outer diameters corresponding to the inner diameter of the outer tube 30 is formed at first. Next, the mandrel is covered by a tube, which is made of thermoplastic resin such as polyamide and has an inner diameter slightly larger than a maximum outer diameter of the mandrel, followed by covering the outer circumference with a heat-shrinkable tube. Then, the heat-shrinkable tube is heated to tighten the thermoplastic resin tube. After cooling, the tube is removed from the mandrel to obtain the outer tube 30 having the first to the third portions. Note that, the outer circumferential face of the outer tube 30 may be polished, if necessary.

In the intra-aortic balloon catheter 20 of the present embodiment, the inner tube 35 is firmly fixed to an inner circumference of the outer tube 30 by an adhesive agent, except for portions with steps on the inner circumference of the outer tube 30. Thereby, a flow channel resistance of the pressurized fluid flowing in the outer tube 30 can be reduced, and the response of the balloon portion 21 can be improved further.

Also, in the intra-aortic balloon catheter 20 of the present embodiment shown in Fig. 1, a distal end opening face 30a of the outer tube 30 is formed so as to take the form of an acute angle to the axial direction of the outer tube 30. Further, at a position spaced with a predetermined width from the distal end opening face 30a of the outer tube 30, an insection. 30b extending to a circumferential direction of the outer tube 30 is formed, a tube wall 30c of the outer tube 30 positioned between the insection 30b and the end opening face 30a is squeezed into the internal portion of the outer tube 30. Thereby, the inner tube 35 is inserted through the expanded insection 30b, wherein the inner tube 35 is engaged to the inner wall of the outer tube 30.

A connector 41 of the intra-aortic balloon catheter 20 is a compact comprising a lumen which opens at three position of an outer tube connection opening 42 as a connection opening of the inner tube 35 and the outer tube 30, a fluid inlet opening 44 as a proximal end opening of a fluid channel for a balloon inflating and a blood pressure taking opening 46 as a proximal end opening of a blood flow channel for the blood pressure taking. A length of the connector 41 is, normally, 10 to 150mm. As a structural material for the connector 41, a thermoplastic resin such as ABS (acrylonitrile butadiene styrene copolymer), polystyrene, polypropylene and polycarbonate is preferably used.

In the intra-aortic balloon catheter 20 shown in Fig. 1, the outer tube 30 is connected to the connector 41 by inserting the proximal end portion of the outer tube 30 in the outer tube connection opening 42, and the inner tube 35 is connected to the connector 41 by inserting the proximal end portion of the inner tube 35 from the outer tube connection opening 42 to the blood pressure taking opening 46 of the connector 41. When using the intra-aortic balloon catheter 20, a pump device for introducing and leading out a fluid such as helium gas in the balloon portion 21 via a tube, for instance, is connected to the fluid inlet opening 44. Then, a blood pressure taking device for measuring the blood pressure via a tube wherein normal saline is filled, for instance, is connected to the blood pressure taking opening 46.

Also, the outer tube connection opening 42 of the connector 41 preferably communicates with the fluid inlet opening 44 by a linear pipe conduit, as shown in Fig. 1. When the outer tube connection opening 42 and the fluid inlet opening 44 communicates by the linear pipe conduit, a flow channel resistance against a fluid for inflating balloon in the connector 41 becomes smaller comparing to the case of using the inflected pipe conduit, so that the response of the balloon portion 21 is improved.

In the intra-aortic balloon catheter 20 shown in Fig. 1, the inner circumference of the proximal end portion of the balloon portion 21 is bonded to the outer circumference of the distal end of the outer tube 30, and the inner circumference of the distal end portion of the balloon portion 21 is bonded to the outer circumference portion of the inner tube 35 via the end tip 40. Thereby, the balloon portion 21 is attached to the outer tube 30 and the inner tube 35. As a bonding method, heat-sealing or adhesive bonding methods may be mentioned. This bond results in maintaining airtight status in an internal portion of the balloon portion 21 to the outer tube 30 except for the distal end opening thereof.

The end tip 40 connected to the distal end portion of the balloon portion 21 is a member which is composed of relatively flexible material, and has its function to prevent the distal end portion of the inner tube 35 from needling a blood vessel wall of the patient. As a material constituting the end tip 40, there may be used natural rubber or synthetic resin such as soft polyvinyl chloride resin, silicon resin, urethane based elastomer, styrene based elastomer, vinyl chloride based elastomer, olefin based elastomer, polyester based elastomer and polyamide based elastomer. However, in view of antithrombogenicity, it is preferable to use polyurethane based elastomer. Also, X-ray contrast agent may be included in the material constituting the end tip 40.

A shape of the end tip 40 is preferably a tubular shape for inserting through a guide wire internally. Also, a distal end of the end tip is preferably hemispherical. An axial length of the end tip 40 is preferably 5 to 25mm, an outer diameter thereof is preferably 1.6 to 3.4mm, and an inner diameter thereof is preferably 0.1 to 1.5mm.

In the intra-aortic balloon pumping set of the present invention, the intra-aortic balloon catheter 20 and the introducer 10 are indispensable components, and other components used in IABP method treatment may be included. As such elements, a dilator, an aseptic sleeve, a guide wire, a puncture needle, a driving tube, a unidirectional valve may be mentioned.

### <Method for Using the Intra-aortic Balloon Pumping Set of the Present Invention>

Next, one example of methods for using the intra-aortic balloon pumping set of the present invention will be described. First, the balloon portion 21 of the intra-aortic balloon catheter 20 was wound around the inner tube 35, and a guide wire is inserted through the inner tube 35 internally. Then, the introducer 10 is needled into a femoral region of the patient by Seldinger method for introducing the distal end of the sheath 1 to the aorta of the patient, and inserting the intra-aortic balloon catheter 20, wherein the guide wire is inserted through, to the aorta of the patient via the introducer 10.

Next, pushing ahead the intra-aortic balloon catheter 20 the balloon portion 21 is placed in the aorta of the patient. Note that most of the first portion 31 of the outer tube 30 project from the distal end of the sheath 1 of the introducer 10 and are placed inside the patient's blood vessel; most of the second portion 32 are placed inside the introducer 10; and whole of the third portion 33 are placed outside the introducer 10 on this occasion.

When the balloon portion 21 is placed in the aorta, the guide wire is removed, a pumping device (not shown) is connected to the fluid inlet opening of the connector 41 via a tube, for example, and a blood pressure taking device (not shown) is connected to the blood pressure taking opening via a tube wherein normal saline is filled, for example. Then, observing the blood pressure change transmitted to the blood pressure taking opening of the connector 41 via the lumen of the inner tube 35 is observed by the blood pressure measuring device, a pumping device is driven based on the observed result, and fluid such as helium gas is introduced in and led out from the balloon portion 21 via a fluid channel. As a result of such operation, the balloon portion 21 is inflated and deflated in accordance with the heartbeat, which supports heart functions.

In the intra-aortic balloon catheter 20 of the intra-aortic balloon pumping set of the present invention, as stated above, it is possible that the outer tube 30 may comprise a largest possible outer diameter in the respective portions in response to the introducer 10 provided in combination. Accordingly, a flow channel cross sectional area of the pressurized fluid channel formed by the lumen of the outer tube 30 can be enlarged and the response of the balloon portion 21 can be improved.

In addition, according to the present invention, the intra-aortic balloon catheter 20 and the introducer 10 are provided as a set. Therefore, without considering specification differences based on different manufacturers of the introducer 10, the outer diameter of the outer tube 30 of the intra-aortic balloon catheter 20 can be enlarged to an allowable maximum limit capable to insert into a specific introducer 10. As a result, the response of the balloon portion 21 is further improved.

Note that the present invention is not limited to the above embodiments and may be variously modified within the scope of the present invention.

Next, the present invention will be explained based on a further specific example.

### [EXAMPLE]

The introducer wherein A=2.73mm, B=3.50mm, C=185mm and the intra-aortic balloon catheter to be set with the introducer were produced as follows.

First, an outer tube made of polyamide was formed by using a metallic mandrel comprising three types of outer diameters and a heat-shrinkable tube, wherein a first portion having an outer diameter of 2.70mm, an inner diameter of 2.36mm and a length of 280mm, a second portion having an outer diameter of 2.80mm, an inner diameter of 2.45mm and a length of 185mm, and a third portion having an outer diameter of 3.70mm, an inner diameter of 3.30mm and a length of 45mm, are continuing.

Also, at the same time or in tandem, there were prepared an inner tube made of polyether ether ketone having an axial length of 720mm, an inner diameter of 0.72mm and a thickness of 0.11mm; a balloon potion made of urethane based elastomer having an axial length of 200mm, an inner volume of 30cc, an outer diameter of 14mm and a film thickness of 70µm; an end tip made of urethane based elastomer having an axial length of 10mm, an inner diameter of 0.72mm and an outer diameter of 2.1mm; and a connector made of ABS. Next, the intra-aortic balloon catheter having the structure shown in Fig. 1 was formed by using the outer tube, the inner tube, the balloon portion, the end tip and the connector.

The outer tube of the intra-aortic balloon catheter was inserted into a simulated blood vessel internally through the introducer, and a first portion 31 of the outer tube was bended three times continuously in half-round with a bending rate of about 5cm in radius. As retaining it bended, helium was introduced in and led out from the balloon portion through an internal portion of the outer tube (pressurized fluid channel) to measure TI, defined as a time that the balloon portion inflates from a minimum deflation condition to a maximum inflation, and TD, defined as a time that the balloon portion deflates from the maximum inflation condition to the minimum deflation condition. As a result, TI+TD was 224msec.

### [COMPARATIVE EXAMPLE]

By using same procedure as the Example, an outer tube was produced wherein a first portion having an outer diameter of 2.70mm, an inner diameter of 2.36mm and a length of 465mm, and a third portion having an outer diameter of 3.70mm, an inner diameter of 3.30mm and a length of 45mm are connected. Forming an intra-aortic balloon catheter as with the Example other than using this outer tube, the response of a balloon portion thereof was measured. As a result, TI+TD is 256msec.

As stated above, to compare the intra-aortic balloon catheter of the Example comprising an outer tube having a second portion with an outer diameter of A to B (mm) and the intra-aortic balloon catheter of the Comparative Example comprising an outer tube not having a second portion with an outer diameter of A t B (mm), the response of the balloon portion of the intra-aortic balloon catheter of the Example was favorable despite using the same introducer 10. Therefore, it was confirmed that a burden on a patient can be lessened and a balloon can be inflated and deflated with a high response according to the intra-aortic balloon pumping set of the present invention.

## Claims

1. An intra-aortic balloon pumping set comprising:
an intra-aortic balloon catheter (20) having a balloon portion (21) insertable inside an aorta and inflating and deflating to assist the heart function, an outer tube (30) with a distal end connected to a proximal end of said balloon portion to introduce pressurized fluid in said balloon portion and guide out from said balloon portion, and an inner tube (35), to which a distal end of said balloon portion is connected, extending through the insides of said balloon portion and said outer tube in the axial direction; and
an introducer (10) used for inserting said balloon portion into a blood vessel, wherein;
said outer tube comprises a first portion (31), a second portion (32) continuously provided at the proximal end of the first portion and a third portion (33) continuously provided at the proximal end of the second portion;
said introducer has a configuration of cylindrical shape into which a tubular member having an outer diameter of B(mm) or smaller can be inserted;
an outer diameter of said first portion is A(mm) or smaller and a length in the axial direction of said first portion is 1 to 400 (mm) ;
an outer diameter of said second portion is A(mm) or larger and less than B (mm) and a length in the axial direction of said second portion is C-10 (mm) to C+30 (mm); and
an outer diameter of said third portion is larger than B(mm); while an inner diameter of an distal end of said introducer is A(mm), and a length in the axial direction of said introducer is C(mm).

2. The intra-aortic balloon pumping set as set forth in claim 1, wherein
the inner diameter of said second portion (32) is larger than the inner diameter of said first portion (31); and
the inner diameter of said third portion (33) is larger than the inner diameter of said second portion (32).

3. The intra-aortic balloon pumping set as set forth in claim 1, wherein
differences in diameters are provided at the boundary portion between said first portion and said second portion and the boundary portion between said second portion and said third portion, respectively; and
the outer diameter of said second portion is substantially uniform along with the axial direction.

4. The intra-aortic balloon pumping set as set forth in claim 1, wherein
the length of said third portion is 1 to 70(mm).

5. The intra-aortic balloon pumping set as set forth in claim 1, wherein
the outer diameter of said first portion is 90% or larger and less than 100% of A (mm) which is the inner diameter of the distal end of said introducer.

## Patentansprüche

1. Intraaortale Ballonpumpenanlage, umfassend:
einen intraaortalen Ballonkatheter (20) mit einem Ballonteil (21), der in das Innere einer Aorta einführbar ist und sich aufbläst und entleert, um die Herzfunktion zu unterstützen, eine äußere Röhre (30) mit einem entfernten Ende, das mit einem nahen Ende des Ballonteils verbunden ist, um eine unter Druck stehende Flüssigkeit in den Ballonteil einzuleiten und sie aus dem Ballonteil herauszuleiten, und eine innere Röhre (35), mit der ein entferntes Ende des Ballonteils verbunden ist, und sich durch die Innenseiten des Ballonteils und der äußeren Röhre in der axialen Richtung erstreckt; und
ein Einführelement (10), das zum Einführen des Ballonteils in eine Blutader benutzt wird, wobei;
die äußere Röhre einen ersten Teil (31), einen zweiten Teil (32), der fortlaufend an dem nahen Ende des ersten Teils bereitgestellt ist, und einen dritten Teil (33), der fortlaufend an dem nahen Ende des zweiten Teils bereitgestellt ist, umfasst;
wobei das Einführelement eine Konfiguration mit einer zylindrischen Form hat, in die ein röhrenförmiges Teil mit einem äußeren Durchmesser von B (mm) oder weniger eingeführt werden kann;
ein äußerer Durchmesser des ersten Teils A (mm) oder weniger ist und eine Länge in der axialen Richtung des ersten Teils 1 bis 400 (mm) ist;
ein äußerer Durchmesser des zweiten Teils A (mm) oder mehr ist und weniger als B (mm) ist und eine Länge in der axialen Richtung des zweiten Teils C-10 (mm) bis C+30 (mm) ist; und
ein äußerer Durchmesser des dritten Teils größer als B (mm) ist; während ein innerer Durchmesser eines entfernten Endes des Einführelements A (mm) ist, und eine Länge in der axialen Richtung des Einführelements C (mm) ist.

2. Intraaortale Ballonpumpenanlage nach Anspruch 1, wobei
der innere Durchmesser des zweiten Teils (32) größer als der innere Durchmesser des ersten Teils (31) ist; und
der innere Durchmesser des dritten Teils (33) größer als der innere Durchmesser des zweiten Teils (32) ist.

3. Intraaortale Ballonpumpenanlage nach Anspruch 1, wobei
Unterschiede in den Durchmessern im Grenzbereich zwischen dem ersten Teil und dem zweiten Teil beziehungsweise dem Grenzbereich zwischen dem zweiten Teil und dem dritten Teil vorgesehen sind; und
der äußere Durchmesser des zweiten Teils im Wesentlichen gleichmäßig entlang der axialen Richtung ist.

4. Intraaortale Ballonpumpenanlage nach Anspruch 1, wobei die Länge des dritten Teils 1-70 (mm) ist.

5. Intraaortale Ballonpumpenanlage nach Anspruch 1, wobei der äußere Durchmesser des ersten Teils 90 % oder mehr und weniger als 100 % von A (mm) ist, der dem inneren Durchmesser des entfernten Endes des Einführelements entspricht.

## Revendications

1. Ensemble de pompage à ballonnet intra-aortique comprenant:
un cathéter à ballonnet intra-aortique (20) ayant une partie à ballonnet (21) pouvant être introduite dans une aorte et se gonflant et se dégonflant pour soutenir la fonction cardiaque, une tube extérieur (30) ayant une extrémité distale reliée à une extrémité proximale de ladite partie à ballonnet pour introduire un fluide sous pression dans ladite partie à ballonnet et pour le conduire hors de ladite partie à ballonnet, et un tube intérieur (35), auquel est reliée une extrémité distale de ladite partie à ballonnet, s'étendant à travers l'intérieur de ladite partie à ballonnet et dudit tube extérieur en direction axiale; et
un introducteur (10) utilisé pour insérer ladite partie à ballonnet dans un vaisseau sanguin, dans lequel ledit tube extérieur comprend une première partie (31),
une deuxième partie (32) prévue en continu à l'extrémité proximale de la première partie, et une troisième partie (33) prévue en continu à l'extrémité proximale de la deuxième partie;
ledit introducteur a une configuration de forme cylindrique dans laquelle un élément tubulaire ayant un diamètre extérieur de B (mm) ou inférieur peut être inséré;
un diamètre extérieur de ladite première partie est A(mm) ou inférieur, et une longueur en direction axiale de ladite première partie est de 1 à 400(mm);
un diamètre extérieur de ladite deuxième partie est A(mm) ou supérieur et inférieur à B(mm), et une longueur en direction axiale de ladite deuxième partie est de C-10 (mm) à C+30 (mm) ; et
un diamètre extérieur de ladite troisième partie est supérieur à B (mm); alors qu'un diamètre intérieur d'une extrémité distale dudit introducteur est A(mm), et une longueur en direction axiale dudit introducteur est C (mm) .

2. Ensemble de pompage à ballonnet intra-aortique selon la revendication 1, dans lequel
le diamètre intérieur de ladite deuxième partie (32) est supérieur au diamètre intérieur de ladite première partie (31); et
le diamètre intérieur de ladite troisième partie (33) est supérieur au diamètre intérieur de ladite deuxième partie (32) .

3. Ensemble de pompage à ballonnet intra-aortique selon la revendication 1, dans lequel
des différences en diamètre sont prévues dans la partie limitrophe entre ladite première partie et ladite deuxième partie et dans la partie limitrophe entre ladite deuxième partie et ladite troisième partie, respectivement; et
le diamètre extérieur de ladite deuxième partie est essentiellement uniforme le long de la direction axiale.

4. Ensemble de pompage à ballonnet intra-aortique selon la revendication 1, dans lequel
la longueur de ladite troisième partie est de 1 à 70(mm).

5. Ensemble de pompage à ballonnet intra-aortique selon la revendication 1, dans lequel
le diamètre extérieur de ladite première partie est 90% ou plus et moins que 100% de A (mm) qui est le diamètre intérieur de l'extrémité distale dudit introducteur.
